# EUROPEAN PATENT SPECIFICATION

(11) **EP 0 724 598 B1**
(45) Date of publication and mention of the grant of the patent: **13.01.1999**
(21) Application number: 94931373.8
(22) Date of filing: 14.10.1994
(51) Int. Cl.: C07K 14/475, C07K 14/48, A61K 38/18

(54) **GROWTH FACTOR HETERODIMERS**
HETERODIMERE DER WACHSTUMSFAKTOREN
HETERODIMERES DE FACTEURS DE CROISSANCE

(30) Priority: 18.10.1993 US 138951
(43) Date of publication of application: 07.08.1996
(73) Proprietor: REGENERON PHARMACEUTICALS, INC., Tarrytown, NY 10591-6707 (US)
(72) Inventor: RADZIEJEWSKI, Czeslaw, N. White Plains, NY 10603 (US)
(74) Representative: Goldin, Douglas Michael
(86) International application number: US9411696
(87) International publication number: WO9511258

(56) References cited:
- EP-A- 0 409 727
- EP-A- 0 450 386
- WO-A-93/18066
- NEUROBIOLOGY, vol. 5, no.6, December 1975 pages 369-381, J.B. MOORE JR. AND E.M. SHOOTER 'The Use of Hybrid Molecules in a Study of the Equilibrium between Nerve Growth Factor Monomers and Dimers'
- JOURNAL OF NEUROCHEMISTRY, vol. 59, no.5, November 1992 pages 1937-1945, L.E. BURTON ET AL. 'Activity and Biospecificity of Proteolyzed Forms and Dimeric Combinations of Recombinant Human and Murine Nerve Growth Factor' cited in the application

## Description

The neurotrophins are small, basic, disulfide-linked dimeric protein factors which have been implicated in the growth and maintenance of neuronal cells [Snider & Johnson, Ann. Neurol. 26:489-506 (1989)]. The neurotrophin family includes nerve growth factor (NGF) as well as brain-derived neurotrophic factor (BDNF), neurotrophin-3 (NT-3) and neurotrophin-4 (NT-4), all of which have recently been molecularly cloned and shown to be members of the NGF family by virtue of their sequence homology [Leibrock, et al., Nature 341: 149-152, (1989); Hohn, et al., Nature, 344: 339-341, (1990); Maisonpierre, et al., Science, 247: 1446-1451, (1990); Rosenthal, et al., Neuron, 4: 767-773, (1990); Ernfors, et al., Proc. Natl. Acad. Sci., U.S.A., 87: 5454-5458 (1990); Jones and Reichardt, Proc. Natl. Acad. Sci. U.S.A. 87:8060-8064 (1990); Hallbook, et al., Neuron 6: 845-858, (1991); Ip, et al., Proc. Natl. Acad. Sci., U.S.A., 89: 3060-3064 (1992)].

This family of proteins plays an important role in both the developing and the adult vertebrate nervous system, where these proteins support survival of various neuronal cell populations, in particular, but not limited to, dopaminergic neurons, cholinergic neurons, sensory neurons, including cranial sensory neurons involved in hearing, taste, vision, balance, etc., cells of the cortex, striatum, hippocampus, cerebellum, olfactory bulbs, periaqueductal gray, raphe nuclei, locus coeruleus, dorsal root Express Mail Certificate No. EF 266651373US ganglion, neural placode derivatives, sympathetic neurons and upper and lower motor neurons. Thus, the neurotrophins are currently being assessed for efficacy in the treatment of motor neuron diseases such as amyotrophic lateral sclerosis (ALS) as well as other neurological disorders such as Parkinson's disease, Alzheimer's disease and epilepsy [Snyder, S. H., Nature 350: 195 (1991); Phillips et al., Neuron 7:695-702 (1991); Enfors et al., Neuron 7:165-176 (1991); published PCT application WO 91/03568 published March 21, 1991; published PCT application WO 91/03569 published March 21, 1991; published PCT application WO 92/22665 published December 23, 1992].

Biological activity of the neurotrophins is mediated by binding to cell surface receptors. Two classes of receptors have been identified: the transmembrane protein p75 and the receptor tyrosine kinases known as the trks. [Lo., Curr. Op. Neurobiol. 2:336-340 (1992); Anderson, Curr. Biol. 2:461-463 (1992)]. The ability of polypeptide ligands to bind cells and thereby elicit a phenotypic response such as cell growth, survival or differentiation in such cells is primarily mediated through the trks, possibly in conjunction with binding to p75. The extracellular portion of each trk is generally the most distinctive portion of the molecule, as it provides the protein with its ligand-recognizing characteristic. Binding of a ligand to the extracellular domain results in signal transduction via an intracellular tyrosine kinase catalytic domain which transmits a biological signal to intracellular target proteins. The particular array of sequence motifs of this cytoplasmic, catalytic domain determines its access to potential kinase substrates [Mohammadi, et al., Mol. Cell. Biol., 11: 5068-5078 (1990); Fantl, et al., Cell, 69:413-413 (1992)].

The receptor and signal transduction pathways utilized by NGF involves the product of the trk proto-oncogene (now known as trkA). [Kaplan et al., Nature 350:156-160 (1991); Klein, et al., Cell 65:189-197 (1991)].

Klein, et al. [EMBO J. 8:3701-3709 (1989)] reported the isolation of trkB, which encodes a second member of the tyrosine protein kinase family of receptors found to be highly related to the human trk protooncogene. TrkB binds and mediates the functional responses to BDNF, NT-4, and, to a lesser extent, NT-3 (Squinto, et al., Cell 65:885-903 (1991); Ip, et al., Proc. Natl. Acad. Sci. U.S.A. 89:3060-3064 (1992); Klein, et al., Neuron, 8:947-956 (1992)]. At the amino acid level, the products of trk and trkB were found to share 57 percent homology in their extracellular regions, including 9 of the 11 cysteines present in trk. This homology was found to increase to 88 percent within their respective tyrosine kinase catalytic domains. The Trk gene family has now been expanded to include the trkC locus, with NT-3 having been identified as the preferred ligand for trkC [Lamballe, et al., Cell 66: 967-979 (1991)].

All known growth factor receptor tyrosine kinases, including the trks, appear to undergo dimerization following ligand binding [Schlessinger, J., Trend Biochem. Sci. 13:443-447 (1988); Ullrich and Schlessinger, Cell, 61:203-212 (1990); Schlessinger and Ullrich, Neuron 9:383-391 (1992)]; molecular interactions between dimerizing cytoplasmic domains lead to activation of kinase function. In some instances, such as the growth factor platelet derived growth factor (PDGF), the ligand is a dimer that binds two receptor molecules [Hart, et al., Science, 240: 1529-1531 (1988); Heldin, J. Biol. Chem. 264:8905-8912 (1989)]. Dimeric growth factor structure may play a functional role in signal transduction. By dimerizing cell surface receptors upon binding, activation of cytoplasmic kinase domains may be accomplished without transmission of conformational changes through a transmembrane domain [Ullrich &Schlessinger, Cell 61:203-212 (1991); Li & Schlessinger, Mol. Cell. Biol. 11:3756-3761 (1991).

Mouse NGF, under physiological conditions, exists as a noncovalent homodimer [Bothwell & Shooter, J. Biol. Chem. 252:8532-8536 (1977)]. The three dimensional structure of mouse NGF has only recently been elucidated by X-ray crystallography [McDonald et al. Nature 345: 411-414 (1991)]. Elongated molecules with three closely spaced disulfide bonds were found to possess novel fold comprised exclusively of β-sheet and loop structures. The X-ray diffraction studies established that the amino acid residues involved in the hydrophobic dimer interface are conserved within the amino acid sequences of the neurotrophins.

At subnanomolar concentrations, human BDNF and human NT-3 also exist as tightly associated homodimers [Radziejewski, et al., Biochemistry 31:4431-4436 (1992)]. Deconvolution analysis of BDNF and NT-3 circular dichroism spectra led to the conclusion that both proteins possess predominantly a β-sheet structure similar to, but also somewhat different from, the structure of NGF. We speculated that the changes in BDNF and NT-3 circular dichroism and fluorescence spectra brought about by denaturation reflected both dimer dissociation and protomer unfolding. Fluorescence depolarization studies of NGF unfolding equilibria in guanidine hydrochloride [Timm & Neet, Prot. Science 1:236-244 (1992)] suggest that NGF homodimers undergo dissociation concomitant with unfolding of the monomers. Other denaturing agents also appear to promote dissociation. Moore and Shooter [Neurobiology 5:369-381 (1975)] utilized low pH to form heterodimers between truncated and full length mouse NGF. Recently, Burton et al. [J. Neurochem. 59:1937-1945 (1992)] provided evidence that the dimeric combination of mouse and human NGF could be obtained by a transient exposure of their mixture to acidic pH [Burton et a. J. Neurochem. 59:1937-1945 (1992)].

Recent X-ray diffraction studies of human transforming growth factor-β (TGF-β) revealed topologies partially resembling that of NGF despite there being little amino acid sequence similarity [Daopin, et al., Science 257:369-373 (1992); Schlunegger & GrÅtter, Nature 385:430-434 (1992); Oefner et al., EMBO J. 11:3921-3926 (1992); Swindells, M. B. Science 258:1160-1161 (1992); Murzin & Chothia, Curr. Opin. Struct. Biol. 2:895-903 (1992)]. In both cases the dimer geometry was shown to be vastly different [McDonald et al., Nature 345:411-414 (1991); Daopin et al., Science 257:369-373 (1992); Schlunegger & GrÅtter, Nature 385:430-434 (1992); Murzin & Chothia, Curr. Opin. Struct. Biol. 2:895-903 (1992)]. Other growth factors have also been found to exist as disulfide linked dimers. Transforming growth factors and members of the platelet derived growth factor family Including platelet derived growth factor and vascular endothelial growth factors exist as covalently linked homodimers or heterodimers [Cheifetz et al., Cell 48:409-415 (1987); Cleason-Welsh et al., Proc. Natl. Acad. Sci. USA 86:4917-4921 (1989); Ogawa, et al., J. Biol. Chem. 267:2325-2328 (1992)]. Recent reports describe the production of heterodimers between BDNF and NT-3 by cells infected with Vaccinia virus coexpressing wild type NT-3 and BDNF (Jungbluth and Barde, Neuroscience Abstracts, 1993, page 248); such heterodimers exhibit substantially less biological activity than mixtures of NT-3 and BDNF.

### SUMMARY OF THE INVENTION

In general, the invention describes the production of stable non-covalent heterodimers of neurotrophins. The invention thus provides isolated and purified heterodimeric neurotrophins comprising a monomeric unit of a first neurotrophin and a monomeric unit of a second neurotrophin. In a particular embodiment the first and second monomeric units are derived from NGF, BDNF, NT-3 or NT-4 or chimeras of said neurotrophins.

The invention further provides pharmaceutical compositions comprising such heterodimeric neurotrophins together with a pharmacologically acceptable carrier or diluent and such heterodimeric neurotrophins for use in the treatment of the human or animal body for therapy or in a method of diagnosis. The invention also relates to the use of such heterodimeric neurotrophins in the manufacture of a medicament for use in the treatment of damage to the nervous system or the treatment of congenital conditions or neurodegenerative disorders or in the treatment of diseases or disorders involving striatal cells or in the treatment of damage or degeneration of striatal or hippocampal cells or in the treatment of motor neuron disorders.

The invention also provides a method for producing a heterodimeric neurotrophin comprising:
a) mixing a homodimeric, first member of the neurotrophin family with a homodimeric, second member of the neurotrophin family;
b) subjecting the mixture to a condition in which dissociation of said first and second member occurs;
c) removing the mixture from said condition such that reassociation and formation of heterodimers occurs; and
d) recovering said heterodimers.

### BRIEF DESCRIPTION OF THE FIGURES

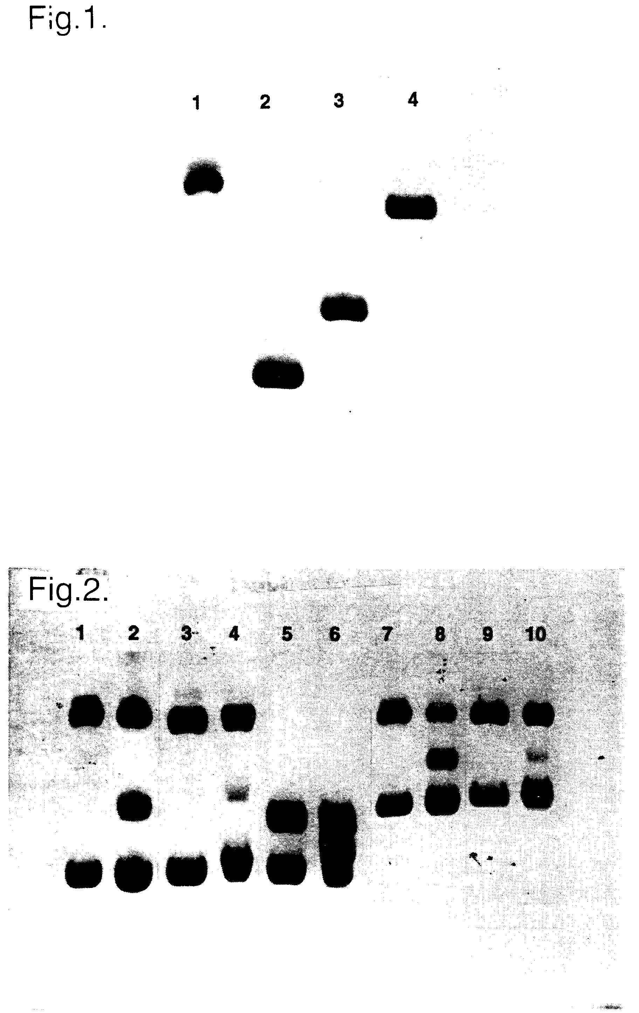

Figure 1. 11% polyacrylamide nondenaturing gel electrophoresis at pH 7.4 of the neurotrophins as described in Materials and Methods. Lane 1: mNGF; lane 2: BDNF; lane 3: NT-3; lane 4 NT-4.

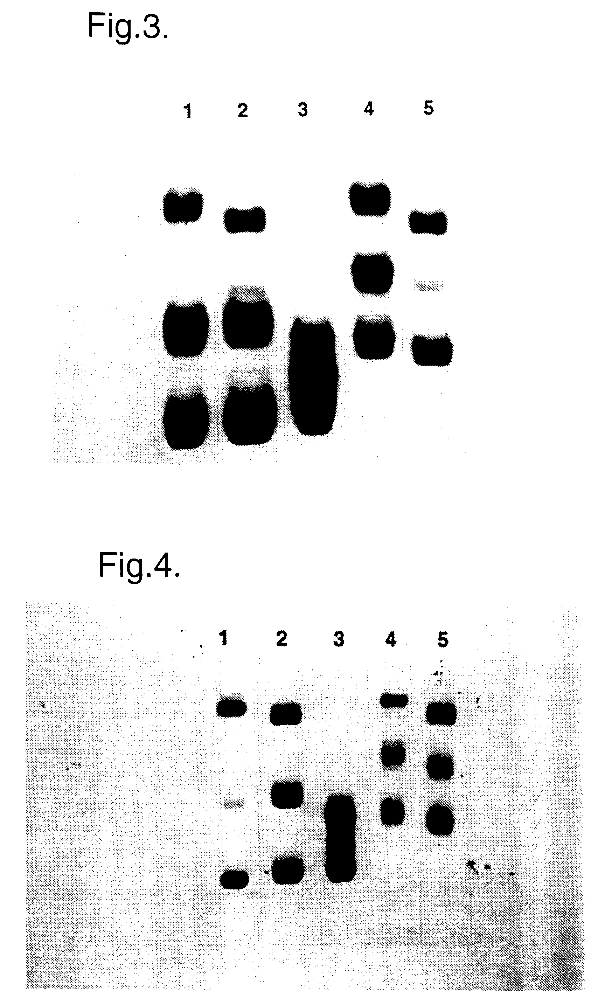

Figure 2. Nondenaturing gel electrophoresis at pH 7.4 of the neurotrophins treated either separately (odd numbered lanes) or jointly (even numbered lanes) with 6 M guanidine hydrochloride followed by the dialysis against neutral buffer. Samples that were treated separately with GuHCl, dialyzed and combined before running the gel are shown in lanes 1, 3, 5 ,7 and 9. Samples that were treated with 6 M guanidine hydrochloride and dialyzed jointly before running the gel are shown in lanes 2, 4, 6, 8 and 10. Lanes 1 and 2: mNGF and BDNF; lanes 3 and 4: NT-4 and BDNF; lanes 5 and 6: NT-3 and BDNF; lanes 7 and 8: mNGF and NT-3; lanes 9 and 10: NT-4 and NT-3. A third band with an intermediate electrophoretic mobility is seen in every even numbered lane.
Figure 3. Nondenaturing gel electrophoresis at pH 7.4 of the neurotrophins treated with 8 M urea and dialyzed against neutral buffer. Only the samples that were treated jointly are shown, how ever control experiment carried with the samples treated separately failed to show the presence of new bands. Lane 1: mNGF and BDNF; lane 2: NT-4 and BDNF; lane 3: NT-3 and BDNF; lane 4: mNGF and NT-3; lane 5: NT-3 and NT-4.
Figure 4. Nondenaturing gel electrophoresis at pH 7.4 of the neurotrophins treated with low pH and dialyzed against neutral buffer as described in Material and Methods. Only the samples that were treated jointly are shown, however control experiment carried with the samples treated separately failed to show the presence of new bands. Lane 1: mNGF and BDNF; lane 2: NT-4 and BDNF; lane 3: NT-3 and BDNF; lane 4: mNGF and NT-3; lane 5: NT-3 and NT-4.

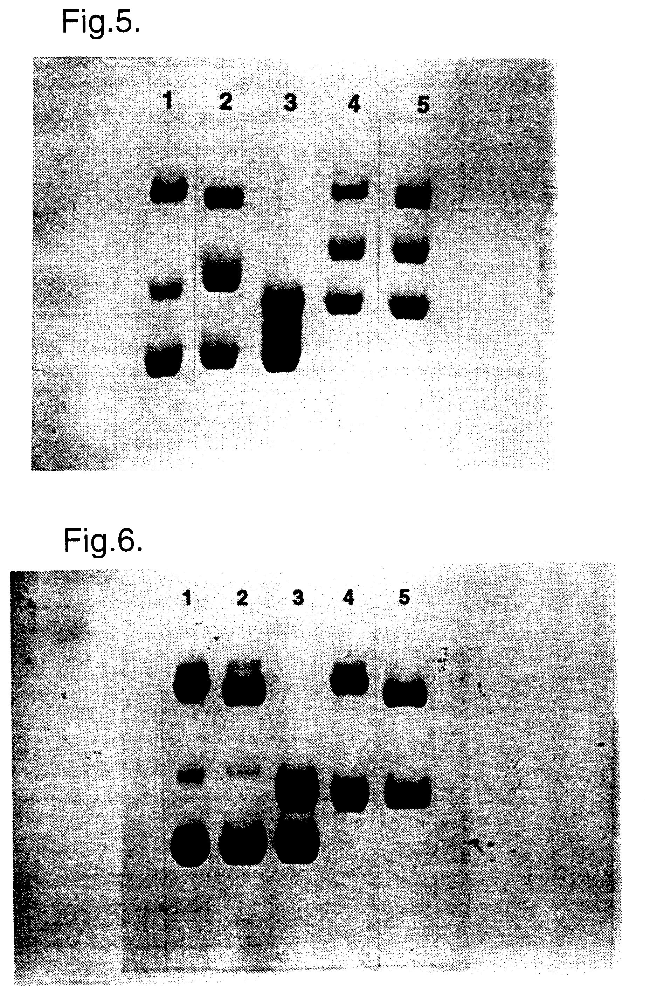

Figure 5. Nondenaturing gel electrophoresis at pH 7.4 of the neurotrophins treated with acetonitrile and dialyzed against neutral buffer as described. Only the samples that were treated jointly are shown, however control experiment carried with the samples treated separately failed to show the presence of new bands. Lane 1: mNGF and BDNF; lane 2: NT-4 and BDNF; lane 3: NT-3 and BDNF; lane 4: mNGF and NT-3; lane 5: NT-3 and NT-4.
Figure 6. Nondenaturing gel electrophoresis at pH 7.4 of the neurotrophins incubated at 37°C for 8 hours in Tris-HCl pH 8. Lane 1: mNGF and BDNF; lane 2: BDNF and NT-4; lane 3: BDNF and NT-3; lane 4: mNGF and NT-3; lane 5 NT-3 and NT-4.

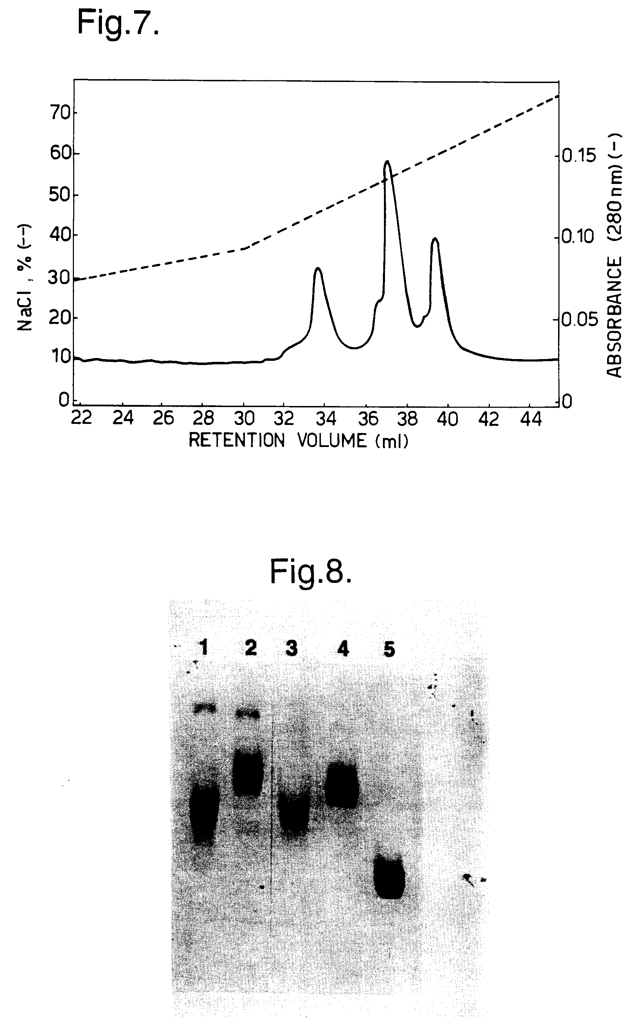

Figure 7. Separation of BDNF/NT-3 heterodimer from the parental homodimers on a Mono S 5x5 cation exchange column using sodium chloride gradient in 20 mM phosphate buffer pH 7. The heterodimer peak eluted between the peak of NT-3 (lower retention volume) and the peak of BDNF (higher retention volume).
Figure 8. 11% nondenaturing polyacrylamide gel electrophoresis of FPLC purified samples of the heterodimers. Lane 1: mNGF/BDNF; lane 2: mNGF/NT-3; lane 3: BDNF/ NT-4; lane 4: NT-3/NT-4; lane 5: BDNF/NT-3. Heterodimers mNGF/BDNF and mNGF/NT-3 were generated by the guanidine hydrochloride method. Heterodimers BDNF/NT-4; BDNF/NT-3 and NT-3/NT-4 were generated by the low pH method.

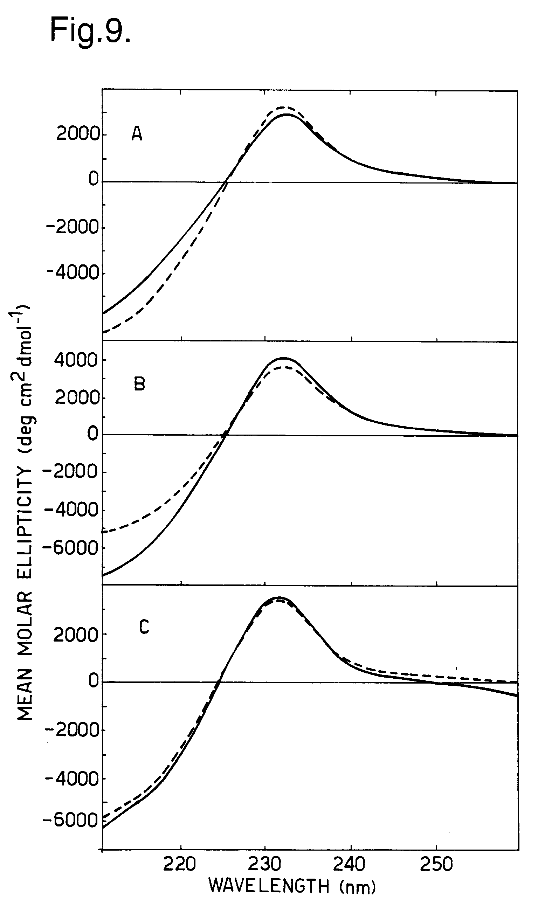

Figure 9. The circular dichroism spectra of the purified heterodimers. a: BDNF/NT-3; b: BDNF/NT-4; c: NT-3/NT-4. The solid line - CD spectra of the heterodimers, dashed line-arithmetical average of the corresponding parental homodimers CD spectra.

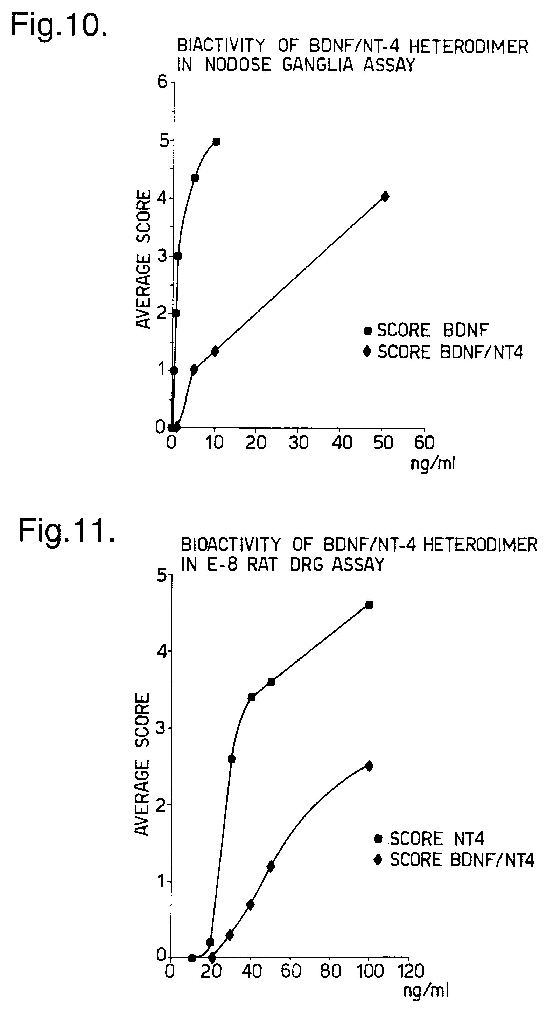

Figure 10. Biological activity of BDNF/NT-4 heterodimer-containing COS supernatants on explanted embryonic day 8 (E8) chick nodose ganglia.
Figure 11. Biological activity of BDNF/NT-4 heterodimer-containing COS supernatants on explanted embryonic day 8 (E8) chick dorsal root ganglia.

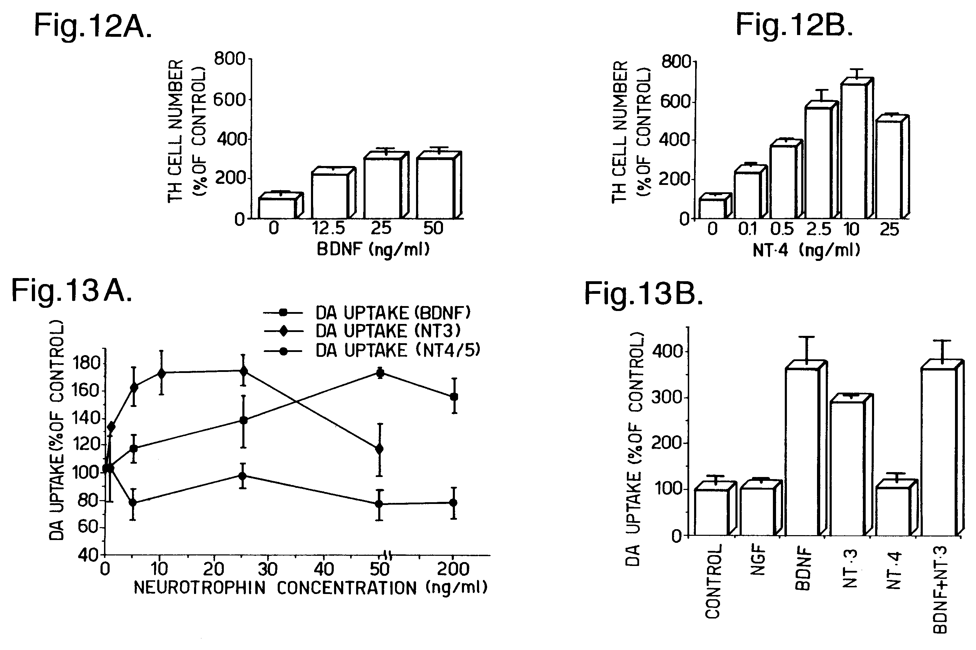

Figure 12. The effect of increasing concentrations of BDNF (Fig 12A) and NT-4 (Fig. 12B) on tyrosine hydroxylase positive dopaminergic neurons of ventral mesencephalon of embryonic day 14 (E14) rat embryos. Data are expressed as a percentage of the TH-positive neurons counted in control cultures (no neurotrophin addition). Results here and in subsequent figures represent the mean ± sd where n=3 or greater.
Figure 13. The effect of treatment with BDNF and NT-4 on high-affinity dopamine uptake activity in cultures of tyrosine hydroxylase positive dopaminergic neurons of ventral mesencephalon of embryonic day 14 (E14) rat embryos. Fig 13A: Dose response curves for BDNF, NT-3 and NT-4. DA uptake activity is expressed as a percentage of the control value measured in untreated cultures. Fig 13B: results obtained from cultures grown in the absence or presence of NGF (50 ng/ml), BDNF (50 ng/ml), NT-3 (25 ng/ml), NT 4/5 (2.5 ng/ml) or a combination of 50 ng/ml BDNF and 12.5 ng/ml NT-3. The cultures were maintained in vitro for a period of 7 days. Results are expressed relative to controls.

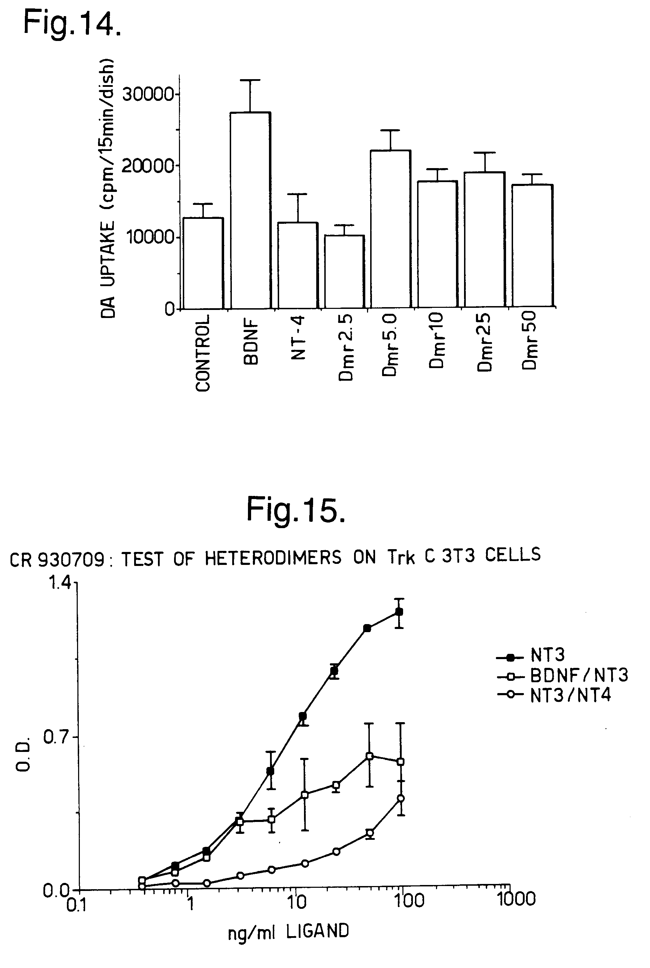

Figure 14. The effect of treatment with BDNF/NT-4 heterodimer on high-affinity dopamine uptake activity in cultures of tyrosine hydroxylase positive dopaminergic neurons of ventral mesencephalon of embryonic day 14 (E14) rat embryos. DA uptake activity is expressed as a percentage of the control value measured in untreated cultures. Results are expressed relative to controls.
Figure 15. Binding of heterodimers to TrkC expressing 3T3 cells. Open squars: BDNF/NT-3; closed squares: NT-3; Open circles:NT-3/NT-4 heterodimer.

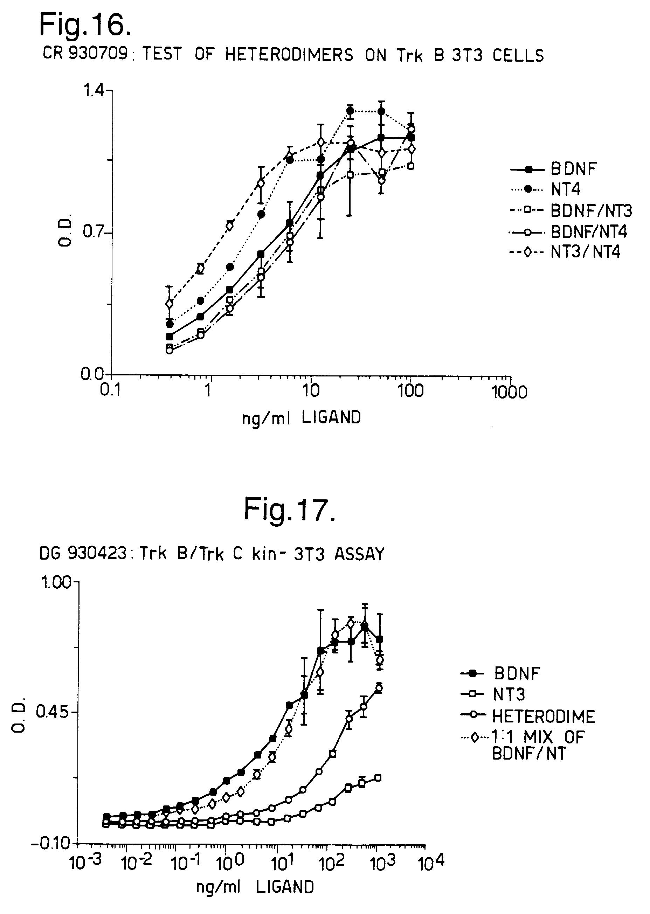

Figure 16. Binding of heterodimers to TrkB expressing 3T3 cells. Open squares: BDNF/NT-3; closed squares: BDNF; Open circles: BDNF/NT-4 heterodimer; closed circles: NT-4; open diamonds:NT-3/NT-4 heterodimer.
Figure 17. Binding of BDNF and NT-3, alone and in combination, as compared to binding of BDNF/NT-3 heterodimers to 3T3 cells coexpressing TrkB and TrkC kin- (TrkC lacking intracellular domain). Closed squares: BDNF; Open squares: NT-3; Open circles: BDNF/NT-3 heterodimer; Open diamonds: 1:1 mix of BDNF and NT-3.

### DETAILED DESCRIPTION OF THE INVENTION

The present invention relates to stable, noncovalent, growth factor heterodimers. Heterodimers produced according to the present invention comprise monomer units derived from growth factors that exist as dimers.

In a preferred embodiment of the invention, a homodimeric, first member of the neurotrophin family is dissociated into its component monomeric units and reassociated in the presence of a second, homodimeric member of the neurotrophin family which has been dissociated into its component monomeric units, to form stable heterodimers comprising a monomeric unit of the first neurotrophin and a monomeric unit of the second neurotrophin. As used herein, "neurotrophin" refers to the dimeric neurotrophic factors that are members of the nerve growth factor (NGF) family. These include NGF, BDNF, NT-3 and NT-4, as well as chimeras thereof, produced as described in U.S. Patent No. 5,169,764 issued on December 8, 1992, as well as mutants or derivatives that have neurotrophin activity, as described, for example, in copending U.S. Patent 5,349,055 issued on September 20, 1994, entitled "Neurotrophic Factors Having Altered Binding Specificities", which is incorporated by reference herein. In addition to the neurotrophins described above, monomeric units derived from other factors which exist as dimers, such as TGF-β and vascular endothelial growth factors, may be used according to the invention to produce novel heterodimers.

Parental dimers that are used to generate monomeric units according to the present invention may be purified from natural tissue, chemically synthesized, or, preferably, produced in recombinant DNA expression systems such as prokaryotic or eukaryotic expression systems, such as, for example, the COS cell system. Methods for producing these molecules include those described in PCT Publication Nos. WO 91/03568, WO 91/03569 and WO 92/20365 which are incorporated by reference herein. They may be isolated using methods, such as affinity chromatography or the like, known by one skilled in the art for the separation of proteins. Recombinant human BDNF and NT-3 are made utilizing available sequence data. (see, for example, for BDNF, published PCT application WO91/03568 published March 21, 1991; for NT-3, published PCT application WO91/03569 published March 21, 1991 and Maisonpierre, et al. Science 247(1990):1446-1451; for NT-4, Ip, et al. Proc. Natl. Acad. Sci. USA 89:3060-3064 (1992)]. Methods for producing these molecules include those described in PCT Publication Nos. WO 91/03568, WO 91/03569 and WO 92/20365.

In a preferred specific embodiment of the invention, BDNF, NT-3 or NT-4 may be isolated from eukaryotic cells (e.g. BDNF transfected Chinese Hamster Ovary (CHO) cells)) expressing the neurotrophin by a two step procedure consisting of cation exchange and gel filtration chromatography. Using BDNF as an example, conditioned medium collected from Opticell bioreactor (Charles River) seeded with cells expressing BDNF may be diluted with distilled water at 1.25:1 ratio and continuously loaded at onto a S-Sepharose Fast Flow (Pharmacia) column. For example, a total volume of 10.6L (diluted) may be applied over a period of about five days to a 15 ml (1.6 cm i.d.) column. The column may be washed with 50 ml of 20 mM MES buffer pH 5.8, followed by 50 ml of the same buffer containing 250 mM sodium chloride followed by the same MES buffer without sodium chloride until absorbance at 280 nm reached baseline level. The column may then be washed with 25 ml of 20 mM bicine buffer pH 8.5 followed by 15 ml of the same buffer containing 100 mM sodium chloride. The column may subsequently be eluted with 150 ml, 100 mM to 750 mM sodium chloride gradient in 20 mM bicine buffer pH 8.5. The flow rate may be about 2.5 ml/min. Absorbance of the eluate may be monitored at 280 nm at a sensitivity of 0.2 absorbance unit full scale. Fractions containing recombinant BDNF may be identified by DRG explant bioassay. The fractions with the highest specific activity may be expected to correspond to a sodium chloride concentration between 500 and 750 mM. These fractions may be combined and concentrated by high pressure membrane ultrafiltration using 3 KDa Omega type membrane, or an equivalent membrane that exhibits low nonspecific binding of BDNF. The concentrated sample may then be applied onto for example, a 120 ml (1.6 cm i.d.) Sephacryl S-100HR gel filtration column (Pharmacia). This column may be packed and equilibrated with 20 mM HEPES buffer pH 7.6 containing 400 mM NaCl. The column may be eluted at 0.25 ml/min. Fractions may be collected and assayed for BDNF activity in the DRG explant bioassay. Fractions eluted between 80 and 90 ml may be expected to contain bioactive recombinant brain derived growth factor. These fractions may be combined and analyzed by reducing SDS polyacrylamide electrophoresis, N-terminal sequencing and quantitative amino acid analysis. Volumes may be adjusted where appropriate. Protein concentrations were calculated from their optical densities at 280 nm. The extinction coefficients of neurotrophin monomers at 280 nm were 18,600 M⁻¹cm⁻¹ for mNGF, 26,800 M⁻¹cm⁻¹ for BDNF, 27,400 M⁻¹cm⁻¹ M for NT-3, and 24,900 M⁻¹cm⁻¹ for NT-4.

According to the invention, dimeric growth factors are mixed and subjected to a condition in which dissociation/unfolding occurs, such as in the presence of a dissociation/unfolding agent, followed by subjection to conditions which allow monomer reassociation and formation of heterodimers. Dissociation/unfolding agents include any agents known to promote the dissociation of proteins. Such agents include, but are not limited to, guanidine hydrochloride, urea, potassium thiocyanate, pH lowering agents such as buffered HCl solutions, and polar, water miscible organic solvents such as acetonitrile or alcohols such as propanol or isopropanol. In preferred embodiments of the invention, the dissociation/unfolding agent is one or more of the following: 6 M guanidine hydrochloride, 8 M urea, low pH buffer (such as HCl/KCl, pH 1.0) or acetonitrile.

In another embodiment of the invention, heterodimers are produced by combining a first and second homodimeric neurotrophin and incubating at 37° C.

Heterodimers may be separated from homodimers using methods available to those skilled in the art. For example, limited quantities of heterodimers may be recovered by passive elution from preparative, nondenaturing polyacrylamide gels. Alternatively, heterodimers may be purified using high pressure cation exchange chromatography. Excellent purification has been obtained using a Mono S cation exchange column.

The formation of heterodimers may be assessed using methods known to those in the art. For example, samples may be run on polyacrylamide, nondenaturing gels.

As described in Examples 2 through 5, heterodimers of neurotrophins appear to retain the activity and/or binding characteristics of one or both parental molecules. Thus, according to the invention, heterodimers can be created that will retain the same utility as the parental molecules, while possessing properties which may be novel. For example, as described in Example 5, BDNF/NT-4 heterodimers appear to retain the biological activity of BDNF with respect to growth and survival of dopaminergic neurons. Upon injection, however, into the lateral ventricle of the brain, the BDNF/NT-4 heterodimer is distributed to a much broader range of tissues than is BDNF, thus offering a BDNF-like molecule that can be utilized at a lower effective concentration than the parental molecule.

In certain embodiments of the invention, heterodimeric protein can be administered to patients in whom the nervous system has been damaged by trauma, surgery, ischemia, infection, metabolic disease, nutritional deficiency, malignancy, or toxic agents. The invention in particular can be used to treat conditions in which damage has occurred to neurons in the basal forebrain, hippocampus or striatum. In addition, it can be used to treat conditions in which damage or degeneration has occurred to spinal sensory neurons, cranial sensory neurons involved in hearing, taste, vision, balance, etc., motor neurons or retinal cells, by administering effective therapeutic amounts of heterodimeric protein or peptide fragments or derivatives. Such uses include, but are not limited to, treatment of retinal detachment, age related or other maculopathies, photic retinopathy, surgery-induced retinopathy, retinopathy of prematurity, viral retinopathy, uveitis, ischemic retinopathy due to venous or arterial occlusion or other vascular disorders, retinopathy due to trauma or penetrating lesions of the eye, peripheral vitreoretinopathy or inherited retinal degeneration.

In further embodiments of the invention, heterodimeric protein can be used to treat congenital conditions or neurodegenerative disorders, including, but not limited to, Alzheimer's disease, Parkinson's disease, Parkinson-Plus syndromes (in which Parkinsonian symptoms result from degeneration of dopaminergic neurons), such as Progressive Supranuclear Palsy (Steele-Richardson-Olszewski Syndrome), Olivoponto- cerebellar Atrophy (OPCA), Shy-Drager Syndrome (multiple systems atrophy), and Guamanian Parkinsonism dementia complex, and Huntington's chorea; in particular, the invention can be used to treat congenital or neurodegenerative disorders associated with sensory nerve dysfunction and degenerative diseases of the retina. For example, the heterodimeric protein, or peptide fragments, or derivatives of the invention can be used in the treatment of hereditary spastic paraplegia with retinal degeneration (Kjellin and Barnard-Scholz syndromes), retinitis pigmentosa, Stargardt disease, Usher syndrome (retinitis pigmentosa with congenital hearing loss), and Refsum syndrome (retinitis pigmentosa, hereditary hearing loss, and polyneuropathy), to name but a few.

In a specific embodiment of the invention, administration of BDNF/NT-4 protein, or peptide fragments or derivatives derived therefrom, can be used in conjunction with surgical implantation of tissue in the treatment of Alzheimer's disease and/or Parkinson's disease. NT-4 and BDNF have been suggested to promote the survival of dopaminergic neurons of the substantia nigra in a dose-dependent manner, supporting the use of the BDNF/NT-4 heterodimer in the treatment of disorders of CNS dopaminergic neurons, including, but not limited to, Parkinson's disease. In addition, NT-4 and BDNF have been observed to sustain the survival of CNS cholinergic neurons and, in particular, basal forebrain cholinergic neurons, indicating that the BDNF/NT-4 heterodimer may be useful in the treatment of disorders involving cholinergic neurons, including, but not limited to Alzheimer's disease. It has been shown that approximately 35% of patients with Parkinson's disease suffer from Alzheimer-type dementia; BDNF/NT-4 produced according to the invention may prove to be a useful single agent therapy for this disease complex. Similarly, heterodimer produced according to the invention may be used therapeutically to treat Alzheimer's disease in conjunction with Down's Syndrome. Heterodimer produced according to the invention can be used in the treatment of a variety of dementias as well as congenital learning disorders.

In another specific embodiment of the invention, the administration of heterodimeric protein or peptide fragments or derivatives derived therefrom can be used for the treatment of diseases or disorders which involve striatal cells, which include, but are not limited to Huntington's chorea, striatonigral degeneration and cerebral palsy. This is based on the fact that certain neurotrophins, such as BDNF and NT-4, support striatal cultures, as indicated by an increase in calbindin immunoreactivity and a high affinity uptake of GABA. A dramatic decrease in calbindin and calbindin mRNA has been detected in the striata of Huntington's chorea patients [Kiyama et al, Brain Res. 525:209-214 (1990); Iacopino, et al, Proc. Natl. Acad. Sci. 87:4078-4082 (1990)].

In another embodiment of the invention, the administration of heterodimeric neurotrophin protein can be used for the treatment of other diseases or disorders which are related to damage or degeneration of striatal or hippocampal cells. Such diseases or disorders may be caused by, for example, stroke, ischemia, hypoglycemia or hypoxia.

In yet another embodiment of the invention, neurotrophin heterodimers may be administered in the treatment of epilepsy-related or other seizures. Reduced levels of the inhibitory transmitter GABA are known to be associated with seizures. For example, high doses of penicillin, which reduce GABA levels, can be used to induce experimental focal epilepsy. Administration of the GABA agonist muscimol into the area of the substantia nigra has been shown to markedly suppress motor and limbic seizures (as measured electrographically) induced by electrical stimulation. McNamara, J. et al., 1984, J. Neurosci. 4:2410-2417. Accordingly, the present invention contemplates use of the neurotrophin heterodimers to enhance levels of GABA and thereby prevent the motor manifestations of seizures.

The present invention provides for methods of treatment of a motor neuron disorder comprising administering, to a patient in need of such treatment, an effective amount of a neurotrophin heterodimer that supports the survival, growth or differentiation of motor neurons.

As used herein, the term motor neuron disorder refers to any condition that disturbs the normal function of motor neurons. Such disorders may or may not specifically or even selectively affect motor neurons. For example, motor neuron disorders that may be treated according to the invention include but are not limited to disorders caused by infarction, infection, exposure to toxin, trauma, surgical damage, degenerative disease or malignancy that may affect motor neurons as well as other components of the nervous system. The methods of the invention may also be directed toward disorders that selectively affect motor neurons such as amyotrophic lateral sclerosis, and including, but not limited to, progressive spinal muscular atrophy, progressive bulbar palsy, primary lateral sclerosis, infantile and juvenile muscular atrophy, progressive bulbar paralysis of childhood (Fazio-Londe syndrome), poliomyelitis and the post polio syndrome, and Hereditary Motor sensory Neuropathy (Charcot-Marie-Tooth Disease).

Effective doses may be determined using methods known to one skilled in the art [see, for example, Fingl, et al., The Pharmacological Basis of Therapeutics, Goodman and Gilman, eds. Macmillan Publishing Co., New York, pp. 1-46 ((1975)]. Effective doses and, if present, toxic doses (no toxic effect has as yet been identified for any known members of the BDNF/NT-3/NGF/NT-4 family) may be determined, preferably in vitro, in order to identify the optimal dose range.

In various particular embodiments of the invention, neurotrophin heterodimers may be administered together with an effective amount of at least one other agent that is, itself, capable of promoting motor neuron survival, growth, or differentiation. For example, heterodimers may be administered together with CNTF. As described in published PCT Application WO 93/01300 published 21 January 1993 entitled "Methods of treatment of motor neuron diseases using members of the BDNF/NT-3/NGF/NT-4 family of molecules", members of the neurotrophin family combined with CNTF has been observed to exert at least an additive effect on motor neuron CAT activity. Accordingly, the present invention contemplates coadministration of a neurotrophin heterodimer and CNTF in the treatment of a motor neuron disorder. The production of CNTF is described in PCT Publication No. WO 91/04316, published April 4, 1991 and incorporated by reference herein.

The heterodimers of the invention may be administered in any pharmacologically acceptable carrier, linked to a carrier or targeting molecule (e.g., antibody, hormone, growth factor, etc.) and/or incorporated into liposomes, microcapsules, and controlled release preparation (including heterodimer expressing cells) prior to administration in vivo.

The administration route may be any mode of administration known in the art, including but not limited to intravenously, intrathecally, subcutaneously, by injection into involved tissue, intraarterially, intranasally, orally, or via an implanted device or cells.

The present invention provides for pharmaceutical compositions comprising a heterodimeric neurotrophin together with CNTF in a pharmacologically acceptable carrier.

Pharmaceutical compositions for use according to the invention include a heterodimeric neurotrophin alone or in combination with, for example, CNTF in a liquid, solid or semi-solid solution. For example, the pharmaceutical composition may comprise one or more of the heterodimeric neurotrophins in an aqueous solution, such as sterile water, saline, phosphate buffer or dextrose solution. Alternatively, the factors may be comprised in a solid (e.g. wax) or semi-solid (e.g. gelatinous) formulation that may be implanted into a patient in need of such treatment.

Administration may result in the distribution of the he odimeric neurotrophin(s) of the invention throughout the body or in a localized area. For example, in some conditions which involve distant regions of the nervous system, intravenous or intrathecal administration of heterodimer may be desirable. Alternatively, and not by way of limitation, when localized regions of the nervous system are involved, such as in motor neuron disorders caused by trauma or surgery, local administration may be desirable. In such situations, an implant containing neurotrophin may be placed in or near the lesioned area. Suitable implants include, but are not limited to, gelfoam, wax, or microparticle-based implants.

In preferred embodiments of the invention, an aqueous solution of factor or factors is administered by subcutaneous injection. Each dose may range from about 0.5 to about 1000 µg per kilogram body weight. When the factor is used in combination with human CNTF, the CNTF dose range may be from about 3 µg to about 30 µg rHCNTF per kilogram of body weight (assuming rHCNTF has an EC₅₀=200 pg/ml, as assayed by Manthorpe, et al., 1986, Dev. Brain Res. 25:191). The dosing schedule for subcutaneous administration of factors may vary from one a week to several daily injections based upon the severity of the disease and the patients sensitivity to the factor(s).

In a preferred embodiment, CNTF is administered subcutaneously three times a week at a dose of between about 10 and 30 µg/kg, with injections of neurotrophin heterodimer injected on alternating days within a dose range of about 100 to about 1000 µg/kg.

Because the progression of ALS has been known to be relentless, it may be necessary to continue to treat patients with the factors described herein for the duration of their life.

### EXAMPLE 1: FORMATION OF NEUROTROPHIN HETERODIMERS

### 1.1 MATERIALS AND METHODS

1.1.1 Proteins. Ultra-pure mouse NGF (mNGF) was purchased from Harlan Bioproducts for Science, Inc. Other neurotrophins were prepared as described above.

1.1.2 Formation of Neurotrophin Heterodimers.
Neurotrophin heterodimers were generated by four different approaches. In each case equimolar amounts of two neurotrophins at a final concentration of approximately 1 mg/ml were mixed and subjected to a treatment promoting dissociation/unfolding followed by dialysis against neutral buffer.

In the first method, two neurotrophins were mixed and dialyzed against 6 M guanidine hydrochloride buffered with Tris-HCl (20 mM, pH 8.0) at room temperature for 6 h. The mixture was then dialyzed at 4°C against two changes of 20 mM Tris HCl pH 8.0 buffer over a 24 h period.

In the second method, heterodimers were made by dialyzing proteins against 8 M urea buffered with Tris-HCl (20 mM, pH 8.0) at room temperature for 8 h and then twice against 20 mM phosphate buffer pH 7.0 at 4°C.

In the third method, a mixture of two neurotrophins was first dialyzed against 135 mM HCl pH 1.0 buffer containing 50 mM KCl at room temperature for 6 h, then at 4°C, against two changes of 20 mM pH 7.0 phosphate buffer over 24 h period. An alternative approach was to incubate three volumes of the same HCl/KCl buffer with one volume of premixed stock neurotrophins for 6 h (all the native neurotrophins were in PBS, the final pH was 1.3), and to dialyze the mixture against 20 mM phosphate buffer pH 7.0.

In the fourth approach, heterodimers were generated with the aid of an organic solvent. Acetonitrile was added to a neurotrophin mixture to reach a final concentration of 45% (v/v). Samples were incubated at room temperature for 6 h then dialyzed at 4°C against Tris-HCl buffer. Higher percentages of acetonitrile were found to cause the neurotrophins to precipitate.

Finally, the formation of some heterodimers was accomplished by incubating an equimolar mixture of the homodimers in 20 mM Tris HCl buffer pH 8.0 at room temperature for a period of at least 24 hours.

1.1.3 Nondenaturing Gel Electrophoresis. The presence of all heterodimers was confirmed by running samples on 11% polyacrylamide, nondenaturing polyacrylamide gels. 10 cm x 8 cm gels without a stacking gel were cast according to an established procedure, [McLellan, T., Anal. Biochem. 126:94-99 (1982)]. Gels cast in 43 mM imidazole, 35 mM HEPES pH 7.4 were found to be optimal for the separation of the neurotrophins and their heterodimers. The electrophoretic separation was run for 120 min at 150 V toward the cathode, and the gels were stained with Coomassie Blue. For the preparative purposes, 1.5 mm, 8% nondenaturing gels were cast at the same pH using a Bio-Rad preparative comb. A small amount of sample (10 µg) was loaded into the reference well for staining and a larger amount of the same sample (250 µg) was loaded into the sample well followed by electrophoresis run at 150 V for 60 min.

1.1.4 Purification of the Heterodimers. Initially, a passive elution from the 8% preparative nondenaturing polyacrylamide gels was employed to separate heterodimers from homodimers. This method allowed the recovery of only limited quantities of the heterodimers.

Cation exchange chromatography on a Pharmacia 5x5 Mono S column was found to be the method of choice to separate heterodimers from homodimers. Eluent buffer A was 20 mM phosphate pH 7.0, and buffer B was 20 mM phosphate buffer containing 1 M NaCl.

Chromatography was performed at room temperature with a typical loading of 0.1-2 mg of protein and a flow rate of 0.75 ml/min. Our elution method was similar to that of Timm and Neet [Prot. Science 1:236-244 (1992)]: 0-15 ml 0%B; 27%B at 20 ml; 37%B at 30 ml; 100%B at 60 ml. The heterodimer peaks were always found to elute between the two corresponding homodimer peaks. Heterodimer fractions were combined and concentrated by ultrafiltration (Amicon ultrafiltration stirred cell Model 3, Filtron Omega 3K membrane). Two changes of 20 mM phosphate buffer pH 7.0 were applied to decrease the salt concentration arising from buffer B.

1.1.5 Circular Dichroism and Fluorescence Spectroscopy. A Jasco J-710 Spectropolarimeter equipped with a computer-based data acquisition system was used to acquire CD spectra. Spectra were obtained at 25°C with a 0.5 mm quartz cell in 20 mM phosphate buffer pH 7.0. In order to get a satisfactory spectra, the protein concentrations used were within the range of 0.2 to 1 mg/ml. For each spectrum, twenty scans were acquired between 200 and 260 nm at 2 nm wavelength intervals. The results were corrected for background and expressed as a mean residue ellipticity. For the heterodimers, the combined molecular weight of two monomers was divided by the combined number of the amino acid residues to give the mean residue mass used in these calculations.

1.1.6 Protein Sequencing. Heterodimer bands were transferred electrophoretically from nondenaturing polyacrylamide gels onto a Porton Hyperbond PVDF membrane. Electroblotting was performed at 90 V for 30 min in a 30 mM histidine, 30 mM MES pH 6 buffer containing 10% methanol. After staining with 0.1% Ponceau S in 5% trichloroacetic acid and destaining in water, protein bands were excised and directly sequenced using a Porton Integrated Microsequencing system.

1.1.7 Stability of the heterodimers. Purified heterodimers were diluted in 20 mM phosphate buffer containing 0.01% Tween 20 to the final concentration of 10 ug/ml and incubated at 37 oC in Nunc-immuno Minisorp test tubes for 24 hours followed by cation exchange chromatography on Pharmacia Mono S 5x5 column. The column was eluted using the above mentioned sodium chloride gradient in 20 mM phosphate buffer pH 7. 10 µg of protein was applied onto the column and the proportion of the homodimers was estimated by peak integration.

1.1.8 Gel filtration chromatography. Gel filtration chromatography was carried out at room temperature on 2 Pharmacia Superose 12 columns connected in series. The excluded and included volumes were 15.5 ml and 40.9 ml respectively. 50-100 µg of protein was injected and eluted at 0.5 ml/min with 20 mM phosphate, pH 7 containing 250 mM sodium chloride.

### 1.2 RESULTS

1.2.1 Formation of Heterodimers. Initial experiments to create heterodimers were executed by treating neurotrophin mixtures with 6 M guanidine hydrochloride. The homodimers were first allowed to dissociate/unfold in the guanidine hydrochloride solution, then to refold/dimerize at pH 8 in Tris-HCl buffer. A monomer from each protein therefore could neither reassociate with a monomer of the same sort, or bind to a monomer of the other neurotrophin. The products were examined by 11%, pH 7.4 native polyacrylamide gel electrophoresis. Figure 1 demonstrates the migration pattern of all four native neurotrophic factors on the nondenaturing 11% polyacrylamide gel. Migration distance of each protein is dependent upon both its pl values (charges) and its size. Despite evident similarities among the neurotrophic factors, their electrophoretic mobilities on 11% nondenaturing gels diverge quite clearly (with the exception of mNGF and NT-4). This figure shows the utility of the nondenaturing gel electrophoresis in the biochemical characterization of the neurotrophins. In Figure 2 even numbered lanes show a third species that resulted from the guanidine hydrochloride treatment of the neurotrophin mixtures. The heterodimeric species migrate half-way between the two native proteins, a behavior expected from a molecule having attributes of both parental neurotrophins. In control experiments, as illustrated in odd numbered lanes, the two neurotrophins were treated separately by the same method and combined just before running the gel. The fact that in this case no new species were formed excluded the possibility of the third band being, for example, a monomer of any parent molecules. Sequencing of protein bands transferred to Hybond PVDF membrane indicated that the bands of intermediate electrophoretic mobility indeed included amino acid sequences of the two starting homodimers, therefore providing unequivocal evidence of the heterodimer formation.

Figure 3 demonstrates the formation of the heterodimers induced by 8 hour incubation of the mixture of two homodimers in 8 M solution of urea, followed by dialysis against neutral buffer. Unlike the incubation in 6 M guanidine hydrochloride, 8 hour incubation in 8 M urea induces no apparent change in the circular dichroism and fluorescence spectra of the neurotrophins but at the same time, is sufficient to generate the heterodimers. It would appear therefore, that the dimer-monomer equilibrium is perturbed in the solution of 8 M urea and the subunit exchange rate is fast enough to result in the formation of heterodimeric species without substantial unfolding. The monomer/dimer equilibrium can also be perturbed by exposing neurotrophic factors to acidic pH. In an acidic environment, protonation of the basic amino acid residues will counteract the adhesive interactions responsible for the stability of the dimeric structure. As demonstrated in figure 4, every possible heterodimer can be generated by exposing a pair of neurotrophins to pH 1 for 6 hours. Similarly, an organic solvent would be expected to weaken intradimeric, hydrophobic interactions and consequently, accelerate the subunit exchange. Figure 5 shows the formation of the heterodimers induced by the presence of acetonitrile. The nondenaturing gels of the neurotrophins treated separately by each of the three methods (urea, low pH, acetonitrile) and combined before the electrophoretic separation showed no new band of an intermediate mobility. The yield of heterodimer formation, estimated by the nondenaturing gel electrophoresis, was to some extent dependent upon a method used to generate that heterodimer. Guanidine hydrochloride method produced high yields of mNGF/BDNF, mNGF/NT-3 and NT-3/BDNF heterodimers but was less suitable for BDNF/NT-4 and NT-3/NT-4. 8 M urea method resulted in high yields of BDNF/mNGF, BDNF/NT-4, BDNF/NT-3 and mNGF/NT-3 but was less efficient for making NT- 3/NT-4 heterodimer. Low pH method resulted in high yields of BDNF/NT-4, BDNF/NT-3, mNGF/NT-3 and NT-3/NT-4 heterodimers but produced lower amounts of BDNF/mNGF. Aqueous acetonitrile method resulted in high yields of BDNF/NT-4, BDNF/NT-3, mNGF/NT-3 and NT-3/NT-4 heterodimers but was less efficient for mNGF/BDNF (Figure 5).

In some cases, the subunit exchange rate was rapid enough to observe the formation of the heterodimers even in the absence of the agents promoting homodimer dissociation. 8 hour incubation at 37°C results in the formation of two heterodimers: BDNF/NT-4 and BDNF/mNGF (Figure 6). A 24 hour incubation of the homodimer mixtures at room temperature also produced the same two heterodimers. A 4 day incubation resulted in the formation of even greater amounts of those two heterodimers. However, no heterodimer was generated as the result of 8 hour incubation at room temperature or 24 hour incubation at 4°C. Therefore, guanidine hydrochloride, urea, acidic buffer and aqueous acetonitrile indeed facilitate subunit exchange.

1.2.2 Purification of the heterodimers . High pressure cation exchange chromatography proved to be the most convenient way to separate the heterodimers from the parental homodimers. Unlike the initially used passive elution from the nondenaturing polyacrylamide gels, this approach allowed us to prepare considerable quantities of the desired heterodimer, sufficient for their characterization. Figure 7 shows the elution profile for the purification of the BDNF/NT-3 heterodimer. It is worth noting that, even in the case of those two very similar growth factors, excellent resolution was achieved with the aid of a Mono S cation exchange column. The degree of separation was considerably better for the other heterodimers. Neither the nondenaturing gel electrophoresis nor the high performance ion exchange chromatography resulted in observable mNGF/NT-4 heterodimer. Purified heterodimers were concentrated and desalted by ultrafiltration and stored at -20°C.

When tested on a nondenaturing polyacrylamide gel, the heterodimers containing NGF showed the presence of their parental homodimers. In contrast, those heterodimers without NGF subunit were essentially homogeneous, indicating their higher stability (Fig.8).

1.2.3 Stability of Heterodimers. Stability of the heterodimers with respect to dissociation and rearrangement to homodimers was examined by reinjecting dilute solutions of heterodimers onto a Pharmacia 5x5 Mono S column. A 24 hour incubation in 20 mM phosphate, pH 7.0 at 37°C of the heterodimers BDNF/NT-4, BDNF/NT-3 and NT-3/NT-4, resulted in a small increase of the appropriate precursor homodimers. For the NT-3/NT-4 heterodimer, the homodimer content increased from 2% to 6%. Likewise for BDNF/NT-4 heterodimer, the homodimer content upon the incubation, increased from 2% to 2.2%. Ion exchange chromatography on a Mono S column failed to detect the presence or any increase in the content of the parental homodimers in the case of BDNF/NT-3 heterodimer. This may be due to a more difficult separation. Most likely, in this case the degree of rearrangement, if any, would be similar to the degree of rearrangement found for BDNF/NT-4 and NT-3/NT-4. In contrast, the injections of purified heterodimers: BDNF/mNGF and mNGF/NT-3, kept at -20°C, showed the presence of the parental homodimers in addition to the heterodimers. Upon incubation at 37°C at 10 µg/ml in the same phosphate buffer, rearrangement process resulted in a significant increase of the content of homodimers. The homodimer content for BDNF/mNGF rose from 12% to 20%. Incubation of the mNGF/NT-3 heterodimer at 37°C resulted in the formation of multiple peaks in the elution gradient on a MonoS column.

1.2.4 Circular dichroism spectra of the heterodimers. The circular dichroism spectra of the freshly purified heterodimers are distinct from the spectra of the homodimers. The CD spectra of the stable heterodimers display maximum of positive ellipticity around 230 nm: NT-3/NT-4 max 231.2 nm; BDNF/NT-3 max232.7 nm; BDNF/NT-4 max231.0 nm (Fig 9). The maxima of the positive ellipticity for the heterodimers fall between the maxima for the parental homodimers. The spectra of the heterodimers were also compared to an arithmetical average of two spectra of the parent two homodimers. The comparison showed that the spectra of NT-3/NT-4, and BDNF/NT-3 heterodimers were close to an average spectra of two corresponding homodimers. In the case of the BDNF/NT-4 heterodimer, the CD spectrum diverge more significantly from such an average. The circular dichroism spectra of the neurotrophic factors in 8 M urea at pH 8 were examined between 260 and 220 nm. Only very small spectral changes were seen after 24 h incubation at room temperature.

1.2.5 Gel filtration chromatography of the heterodimers. Size exclusion chromatography on two Superose 12 columns connected in series allowed us to obtain a baseline separation of NT-4 from BDNF, NT-4 from NT-3 as well as partial separation of NT-3 from mNGF. As expected, the elution volumes for the heterodimers fall between those for the homodimers. The elution volumes for NT-4, BDNF/NT-4 heterodimer and BDNF were 27.97 ml, 30.5 ml and 31.28 ml respectively. The elution volumes for NT-3/NT-4 heterodimer and NT-3 were 29.12 ml and 30.34 ml respectively. These findings also provide further evidence that the observed species are indeed heterodimers derived from the starting homodimers and excludes a possibility of the new species being, for example, complexes of the homodimers. Furthermore, the stoichiometry of association in the heterodimers must be 1:1. High performance gel filtration in addition to cation exchange chromatography may be used to further purify small quantities of neurotrophin heterodimers.

### EXAMPLE 2: HETERODIMERS SUPPORT THE OUTGROWTH OF NODOSE GANGLIA AND DORSAL ROOT GANGLIA EXPLANTS

### 2.1 MATERIALS AND METHODS

Biological activity of COS supernatants was determined by measuring the responses of explanted embryonic day 8 (E8) chick nodose ganglia (NG) and dorsal root ganglia (DRG) to increasing doses of BDNF/NT-4 heterodimer. Ganglia were cultured for the times indicated as explants in 1 ml of collagen gel, as described in Lindsay and Rohrer [Devel. Biol. 112:30-48 (1985)]. Fiber outgrowth was scored on a scale of 0 to 5, where 5 is the maximum fiber outgrowth observed on dorsal root ganglia in response to a saturating dose of nerve growth factor (NGF) (1-10ng/ml). BDNF/NT-4 heterodimer was prepared as described above.

### 2.2 RESULTS

Experiments using the BDNF/NT-4 heterodimer provided proof that the heterodimers retain the biological activity of the parental molecules. As shown in Figures 10 and 11, both DRG's and NGs responded to the BDNF/NT-4 heterodimer. Based on these results, the NT-4/BDNF heterodimer would be expected to have at least the same therapeutic properties as BDNF and/or NT-4.

### EXAMPLE 3: BDNF/NT-4 HETERODIMER SUPPORTS THE GROWTH AND SURVIVAL OF DOPAMINERGIC NEURONS

Although BDNF and NT-4 are capable supporting a wide variety of neuronal cell populations, their activities appear to diverge with respect to their effects in promoting the survival and/or regulation of expression of phenotypic markers of dopaminergic neurons present in dissociated cultures of embryonic rat ventral mesencephalon. Accordingly, these experiments were designed to determine whether the BDNF/NT-4 heterodimer had the activity of both parent neurotrophins. Dopaminergic neuron number and phenotypic expression were monitored by tyrosine hydroxylase (TH) immunocytochemistry, and measurement of high-affinity dopamine uptake activity and dopamine content, respectively.

### 3.1 MATERIALS AND METHODS

3.1.1 Preparation of Ventral Mesencephalon Cultures from E14 Rat. Cultures were prepared from the ventral mesencephalon of embryonic day 14 (E14) rat embryos as before [Hyman, et al, Nature 350:230-232 (1991); Spina et al., J. Neurochem 59:99-106 (1992)]. The single cell suspension obtained following trypsinization and mechanical dissociation of the brain tissue was seeded at a density of 5 x 10⁴ cell per cm² onto 35mm dishes which had been precoated with poly-L-lysine and merosin (Collaborative Research). After a 4 hour incubation in MEM supplemented with glutamine (2mM), glucose (6 mg/ml), penicillin G (0.5U/ml), streptomycin (5 µg/ml), and fetal calf serum (FCS,7.5%) to allow for cell attachment to the substratum, cells were cultured in the presence or absence of trophic factors in a defined medium consisting of F12 and basal Eagle medium (1:1,v/v) with N₂ supplements as described by Bottenstein and Sato, Proc. Natl. Acad. Sci. USA 76:514-517 (1979), except that the insulin concentration was reduced to 20 ng/ml, and glutathione was included at 2.5 µg/ml.

3.1.2 ³H-dopamine Uptake Measurement The measurement of high-affinity dopamine (DA) uptake activity was carried out according to the method of Tomozawa and Appel, Brain Res. 399:111-124 (1986). In each treatment group for which high-affinity DA uptake was to be measured, groups of 6 plates were used. Triplicate cultures were assayed for uptake of ³H-dopamine in the presence of 5 µM benztropine to determine non-specific uptake, and three further cultures were assayed under identical conditions except for the omission of benztropine, in order to assess total uptake. Uptake was carried out using ³H-dopamine (NEN, 40 Ci/mmol) at a final concentration of 50nM, for 15 minutes. Specific uptake was calculated as the difference between total uptake and that intake which occurred in the presence of 5 µM benztropine. The non-specific uptake represented between 10-15% of the total uptake in the cultures.

3.1.3 Measurement of Dopamine Content Dopamine content was determined by an adaptation of the method of Refshauge et al, Life Sci. 14:311-322 (1974) and DiPaulo, et al, J. Can. Sci. Neurol., 9:421-427 (1982). Cultures were prepared and maintained in vitro for various times, either in the presence or absence of trophic factors. At the end of the culture period, cells were harvested and immediately acidified with 0.4 N perchlorate containing 0.1 mM ascorbate and 2.5 ng/ml dihydroxybenzylamine (DHBA, an internal standard). Following homogenization and centrifugation to remove precipitated protein, the catecholamines in the extract were absorbed onto alumina (ICN). The samples were then washed extensively, and the catecholamines recovered by extraction from the alumina with 174nM acetic acid containing 0.05% sodium disulfite and 0.025% EDTA. The protein content of the sample was determined after resuspension of the pelleted precipitate in PBS by the method of Smith et al, Anal. Biochem. 76-85 (1985). The catecholamine extracts were injected into the HPLC system (Waters 600E) and separated using a reverse phase C₁₈ column by isocratic elution in a mobile phase consisting of the following: 0.01 M NaPO₄, 1.0 mM EDTA, 0.05mM sodium octyl sulfate, pH 3.0. Quantitation of the various catecholamines was carried out after electrochemical detection (ESA 5100A detector) of the peaks eluted from the HPLC, and the data was normalized to the protein content of the sample.

3.1.3 Tyrosine Hydroxylase (TH) Immunocytochemical Staining. Cultures to be stained for TH containing neurons were fixed in 4% paraformaldehyde and preincubated in phosphate buffer containing 2% horse serum (Vector labs, Malverne, PA). After a brief incubation in phosphate buffer containing 0.02% saponin to permeabilize the cells, cultures were incubated overnight at 4°C with mouse anti-rat TH IgG at 1.0 µg/ml in phosphate buffer, containing 1.5% horse serum and 0.02% saponin. Following rinses to remove unbound primary antibody, the cells were incubated with horse anti-mouse IgG conjugated to biotin (Vector Labs, Malverne, PA) at a concentration of 15 ug/ml. Specifically bound antibody was subsequently visualized following incubation with avidin-horseradish peroxidase (HRP) (Vector Labs ABC reagent) and diaminobenzidine (DAB).

### 3.2 RESULTS

The dose response data shown in Fig. 12 demonstrate that BDNF and NT-4 exerted their effects on dopaminergic neurons in a dose-dependent, saturable manner. NT-4 had a dramatic effect on dopaminergic neuronal survival, producing an almost 7-fold increase in TH-positive neurons after one week in vitro. In the experiment shown in Fig. 12, NT-4 was replenished as multiple every other day additions; similar treatment with BDNF over 8 days in vitro produced a maximal 5-fold increase in TH-positive neurons (Hyman, et al, Nature 350:230-232 (1991).

Increased dopamine uptake activity produced by BDNF treatment was shown to be dose-dependent and saturable (Fig.13). The effect of BDNF was in agreement with previous findings of Knüsel, et al [J. Neurosci. 10:558-570 (1991)]. Differences between NT-4 and BDNF become apparent, however, when examining DA uptake activity. Despite its ability to elevate the number of TH-positive neurons, NT-4 had no effect on high-affinity DA uptake activity. Thus, the effects of NT-4 on the dopaminergic neuron population are apparently distinct as well as overlapping with that observed for BDNF. This suggests that although BDNF and NT-4 show equivalence as TrkB ligands when this receptor is expressed in the artificial setting of a fibroblast [Glass et al., Cell 66:405-413 (1991); Ip et al., Neuron 10:1-10 (1993); Ip, et al., Proc. Natl. Acad. Sci. USA 89:3060-3064 (1992)], in the context of TrkB expressing neurons, the signal transduction pathways of BDNF and NT-4 may be indistinguishable.

The effect on DA uptake was used to determine whether the BDNF/NT-4 heterodimer exhibited BDNF- or NT-4-like activity. The results of DA-uptake experiments on ventral mesencephalon cultures of embryonic day 14 (E14) rat embryos are shown in Figure 14. Saturating concentrations of BDNF (50ng/ml) and NT-4 (2.5 ng/ml) were used, based on the dose response curves for BDNF and NT-4 shown in Figure 13. As shown in Figure 14, the BDNF/NT-4 heterodimer exhibited uptake characteristics that resembled BDNF, rather than NT-4, indicating that the heterodimer exhibited the biological activity of BDNF with respect to this culture system.

### EXAMPLE 4. ACTIVITY OF HETERODIMERS ON TRK RECEPTORS

In this survival assay system, 3T3 fibroblasts, which do not express neurotrophin receptor proteins, have been transfected with trkA, a proto-oncogene encoding a tyrosine kinase receptor for NGF, with trkB, a tyrosine kinase which serves as a functional binding protein for BDNF and NT-3, or with trkC, a tyrosine kinase which serves as a functional binding protein for NT-3. The transfected cells are dependent upon the addition of the corresponding neurotrophin for survival, and thus may be used to assay for biological activity of neurotrophins.

Heterodimers produced as described above were examined in survival assays utilizing 3T3 fibroblasts. To determine the biological activity of BDNF/NT-3 heterodimers, proliferation assays were performed on fibroblasts expressing either TrkB, TrkC, or co-expressing TrkB and an inactive, truncated form of TrkC (these cells are labelled TrkB/TrkCkin- cells). We have previously shown the following: BDNF shows proliferation activity on TrkB cells but not TrkC-expressing fibroblasts, whereas NT-3 shows activity on both TrkB and TrkC cells, though it is less potent on TrkB than BDNF.

4.1 Materials and Methods. Full-length rat trkB and trkC cDNA clones were obtained by screening a rat brain cDNA library in the lambda ZAP2 vector (Stratagene) with rat trkB-specific and trkC specific oligonucleotides corresponding to the most 5' and 3' coding regions of trkB and trkC. The rat trkB and trkC cDNAs were subcloned into pCMX to generate pCMX-trkB and pCMX-trkC.

3T3 fibroblasts were cultured and transfected with pCMX-trkB or trkC as described in Glass, et al., Cell 66:405-413 (1991).

The following heterodimers were tested on both trkB and trkC expressing NIH 3T3 cells: BDNF/NT-3; BDNF/NT-4; and NT-3/NT-4.

### 4.2 RESULTS

4.2.1 BDNF/NT-3 and NT-3/NT-4 Heterodimers Bind TrkB and TrkC. As shown in Figures 15 and 16, both of these heterodimers that comprise a monomer derived from NT-3, which preferentially binds the TrkC receptor, maintained the ability to bind to this receptor as expressed by NIH 3T3 cells. Further, both of these heterodimers comprise a monomer that preferentially binds trkB (BDNF and NT-4). Both of these heterodimers maintained their ability to bind TrkB.

These results indicate that the formation of the heterodimers described herein does not affect the ability of the component monomers molecules to bind to the Trk receptors.

Interestingly, when these neurotrophins are tested on the TrkB/TrkCkin- cells which are lacking the intracellular domain of TrkC, BDNF is as active as on TrkB cells, indicating that it is not bound by the inactive TrkCkin- receptor (Figure 17). In contrast, NT-3 demonstrates greatly reduced activity on the TrkB/TrkCkin-cells in comparison to its activity on the TrkB cells, indicating that it has a "preference" for the TrkC receptor, which is nonfunctional in these cells.

Given the ability of the TrkB/TrkCkin- line to distinguish between the neurotrophins' binding ability on TrkB versus TrkC, we tested to see the effect of BDNF/NT-3 heterodimers on these cells. As shown in Figure 17, the heterodimeric neurotrophin activates TrkB/TrkCkin- cells better than NT-3 does, indicating that the TrkCkin- receptor competes for the heterodimeric neurotrophin less efficiently than it competes NT-3. As a control, BDNF and NT-3 were mixed at a 1:1 ratio to determine if the BDNF:NT-3 mix was similar in activity to the BDNF/NT-3 heterodimer. We saw a significant difference in activities in the mix as compared to the heterodimer, consistent with the idea that the heterodimer constitutes a novel molecule with a unique binding "face" for each Trk receptor.

Further evidence that the heterodimeric molecules have unique binding characteristics was found by comparing BDNF/NT-3 heterodimers to NT-4/NT-3 heterodimers. Both BDNF and NT-4 are inactive on TrkC, whereas NT-3 is the "preferred" ligand for TrkC. If the formation of heterodimers simply diluted the NT-3 binding capability by 1/2, than BDNF/NT-3 heterodimers should have similar activating abilities as NT-4/NT-3 heterodimers. However when these two heterodimers were compared for proliferation activity on TrkC cells (Figure 15), they were found to be clearly different, with the BDNF/NT-3 heterodimer having an E50 of 10ng/ml whereas the NT-4/NT-3 heterodimer has an E50 of approximately 60 ng/ml. 57.

### EXAMPLE 5. DISTRIBUTION OF HETERODIMER IN BRAIN TISSUE

The distribution of BDNF, NT-4 and BDNF/NT-4 heterodimer in brain tissue was measured after a single injection into the lateral ventricle of the brain.

### 5.1 MATERIALS AND METHODS

Equivalent amounts of BDNF, NT-4 or the heterodimer (10 µg in 12 µl buffered solvent) were injected into the lateral ventricle of 3 groups of rats. The injections were made with a Hamilton syringe. Stereotaxic surgery was performed to lower the Hamilton syringe needle through a hole in the skull and into the lateral ventricle. The injection was made over a 12 minute time period. The rats were sacrificed 20-24 hours later by giving a large dose of anesthetic and then perfusing them through the heart with paraformaldehyde fixative. The brains were processed for immunohistochemical localization of the exogenous neurotrophins. The BDNF was labeled by antisera raised against BDNF in turkeys which does not crossreact with NT-4). The NT-4 was labeled by an antiserum raised against NT-4 in a chicken (Regeneron RGC 09; this antiserum does not crossreact with BDNF). The heterodimer was labeled by the anti-BDNF antisera in one set of sections, and by the anti-NT-4 antiserum in an adjacent set of sections from the same brains. Both antisera gave a similar staining pattern for the heterodimer, although the staining with the anti-NT-4 was darker than that with the anti-BDNF. The sensitivity of the two antisera in detecting their respective antigens or the heterodimer is similar as determined in nitrocellulose slot blots of various quantities of neurotrophins.

### 5.2 RESULTS

Inspection of the immunohistochemically stained sections indicated that BDNF penetration from the lateral ventricle into the brain tissue was distinctly more limited than that of NT-4 or the NT-4/BDNF heterodimer. The distances of penetration for NT-4 and the heterodimer were similar. Near the injection site, the distance that the staining extended from the wall of the lateral ventricle into the brain tissue was as follows: BDNF, 0.3 mm; NT-4, 0.76 mm; heterodimer, 0.63 mm. The exogenous neurotrophin staining also was present in the brain tissue surrounding the rest of the ventricular system, and in the brain tissue on the ventral surface of the brain. In the brain tissue surrounding all ventricles, the penetration of the NT-4 and the heterodimer into the brain was clearly greater than that of BDNF.

### 5.3 DISCUSSION

These results provide evidence of a BDNF/NT-4 heterodimer that exhibits the biological properties of BDNF and the more desirable delivery properties of NT-4, thus illustrating the potential of neurotrophin heterodimers that have improved properties over the parental neurotrophin molecules. As shown in EXAMPLE 3, BDNF/NT-4 heterodimers exhibit biological properties characteristic of BDNF. Brain tissue distribution studies shown in this section provide evidence, however, that the BDNF/NT-4 heterodimer will reach larger portions of the brain than will an equivalent amount of BDNF when these substances are injected into the lateral ventricle. Thus, this data demonstrates that the creation of heterodimers may provide a mechanism of enhancing the delivery properties of a neurotrophin, such as BDNF, that has desirable biological properties.

## Claims

1. A method for producing a heterodimeric neurotrophin comprising:
a) mixing a homodimeric, first member of the neurotrophin family with a homodimeric, second member of the neurotrophin family;
b) subjecting the mixture to a condition in which dissociation of said first and second member occurs;
c) removing the mixture from said condition such that reassociation and formation of heterodimers occurs; and
d) recovering said heterodimers.

2. A method according to claim 1 wherein said first and second members are selected from NGF, BDNF, NT-3, NT-4 or chimeras of said neurotrophins.

3. A method according to claim 1 or 2 wherein said dissociation condition is the presence of a dissociation/unfolding agent.

4. A method according to claim 3 wherein said dissociation/unfolding agent is selected from guanidine hydrochloride, urea or acetonitrile.

5. A method according to claim 1 or 2 wherein said dissociation condition is low pH.

6. A method according to any one of the preceding claims wherein said recovery is by passive elution from preparative, non-denaturing polyacrylamide gels.

7. A method according to any one of claims 1 to 5 wherein said recovery is by high pressure cation exchange chromatography.

8. A method according to claim 7 wherein said cation exchange is a Mono S cation exchange column.

9. An isolated and purified heterodimeric neurotrophin comprising a monomeric unit of a first neurotrophin and a monomeric unit of a second neurotrophin.

10. A heterodimeric neurotrophin according to claim 9 wherein said first and second monomeric units are derived from a neurotrophin selected from NGF, BDNF, NT-3 OR NT-4 or chimeras of said neurotrophins.

11. A pharmaceutical composition comprising a heterodimeric neurotrophin according to claim 9 to 10 together with a pharmacologically acceptable carrier or diluent.

12. A heterodimeric neurotrophin according to claim 9 to 10 for use in the treatment of the human or animal body for therapy or in a method of diagnosis.

13. A heterodimeric neurotrophin according to claim 12 for use in the treatment of damage to the nervous system or the treatment of congenital conditions or neurodegenerative disorders or in the treatment of diseases or disorders involving striatal cells or in the treatment of damage or degeneration of striatal or hippocampal cells or in the treatment of motor neuron disorders.

14. Use of a heterodimeric neurotrophin according to claim 9 or 10 in the manufacture of a medicament for use in the treatment of damage to the nervous system or the treatment of congenital conditions or neurodegenerative disorders or in the treatment of diseases or disorders involving striatal cells or in the treatment of damage or degeneration of striatal or hippocampal cells or in the treatment of motor neuron disorders.

## Patentansprüche

1. Verfahren zur Herstellung eines heterodimeren Neurotrophins, umfassend:
a) Mischen eines homodimeren ersten Mitglieds der Neurotrophinfamilie mit einem homodimeren zweiten Mitglied der Neurotrophinfamilie,
b) Aussetzen des Gemisches einem Zustand, in dem die Dissoziation des ersten und zweiten Mitglieds stattfindet;
c) Entfernung des Gemisches aus dem Zustand, so daß Reassoziation und Bildung der Heterodimeren stattfindet; und
d) Gewinnung der Heterodimeren.

2. Verfahren nach Anspruch 1, wobei das erste und zweite Mitglied ausgewählt sind aus NGF, BDNF, NT-3, NT-4 und Chimären der Neurotrophine.

3. Verfahren nach Anspruch 1 oder 2, wobei der Dissoziationszustand die Gegenwart eines Dissoziations/Entfaltungsmittels ist.

4. Verfahren nach Anspruch 3, wobei das Dissoziations/Entfaltungsmittel ausgewählt ist aus Guanidinhydrochlorid, Harnstoff oder Acetonitril.

5. Verfahren nach Anspruch 1 oder 2, wobei der Dissoziationszustand ein niedriger pH-Wert ist.

6. Verfahren nach einem der vorhergehenden Ansprüche, wobei die Gewinnung durch passive Elution aus präparativen nicht-denaturierenden Polyacrylamidgelen erfolgt.

7. Verfahren nach einem der Ansprüche 1 bis 5, wobei die Gewinnung durch Hochdruckkationenaustausch-Chromatographie erfolgt.

8. Verfahren nach Anspruch 7, wobei der Kationenaustausch an einer Mono S-Kationenaustauschersäule erfolgt.

9. Isoliertes und gereinigtes heterodimeres Neurotrophin, das eine monomere Einheit eines ersten Neurotrophins und eine monomere Einheit eines zweiten Neurotrophins umfaßt.

10. Heterodimeres Neurotrophin nach Anspruch 9, wobei die erste und zweite monomere Einheit abgeleitet sind von einem Neurotrophin, das ausgewählt ist aus NGF, BDNF, NT-3 oder NT-4 und Chimären der Neurotrophine.

11. Arzneimittel, umfassend ein heterodimeres Neurotrophin nach Anspruch 9 oder 10 zusammen mit einem pharmakologisch verträglichen Träger oder Verdünnungsmittel.

12. Heterodimeres Neurotrophin nach Anspruch 9 oder 10 zur Verwendung bei der Behandlung des menschlichen oder tierischen Körpers zur Therapie oder in einem Diagnoseverfahren.

13. Heterodimeres Neurotrophin nach Anspruch 12 zur Verwendung bei der Behandlung einer Schädigung des Nervensystems oder der Behandlung von angeborenen Leiden oder neurodegenerativen Störungen oder der Behandlung von Krankheiten oder Störungen, die Striatumzellen betreffen, oder der Behandlung einer Schädigung oder Degeneration von Striatum- oder Hippocampuszellen oder der Behandlung von Motoneuronen-Störungen.

14. Verwendung eines heterodimeren Neurotrophins nach Anspruch 9 oder 10 zur Herstellung eines Arzneimittels zur Verwendung bei der Behandlung einer Schädigung des Nervensystems oder der Behandlung von angeborenen Leiden oder neurodegenerativen Störungen oder der Behandlung von Krankheiten oder Störungen, die Striatumzellen betreffen, oder der Behandlung einer Schädigung oder Degeneration von Striatum- oder Hippocampuszellen oder der Behandlung von Motoneuronen-Störungen.

## Revendications

1. Procédé pour la préparation d'une neurotrophine hétérodimère, comprenant:
a) le mélange d'un premier membre homodimère de la famille des neurotrophines avec un second membre homodimère de la famille des neurotrophines;
b) la soumission du mélange à une condition dans laquelle se produit la dissociation dudit premier membre et dudit second membre;
c) la soustraction du mélange de ladite condition de sorte qu'il se produit une réassociation et une formation d'hétérodimères; et
d) la récupération desdits hétérodimères.

2. Procédé selon la revendication 1, dans lequel lesdits premier et second membres sont choisis parmi NGF, BDNF, NT-3, NT-4 et des chimères desdites neurotrophines.

3. Procédé selon la revendication 1 ou 2, dans lequel ladite condition d'association est la présence d'un agent de dissociation/déploiement.

4. Procédé selon la revendication 3, dans lequel ledit agent de dissociation/déploiement est choisi parmi le chlorhydrate de guanidine, l'urée et l'acétonitrile.

5. Procédé selon la revendication 1 ou 2, dans lequel ladite condition de dissociation est un pH bas.

6. Procédé selon l'une quelconque des revendications précédentes, dans lequel ladite récupération est effectuée par élution passive de gels préparatifs de polyacrylamide non dénaturants.

7. Procedé selon l'une quelconque des revendications 1 à 5, dans lequel ladite récupération est effectuée par chromatographie d'échange de cations à haute pression.

8. Procédé selon la revendication 7, dans lequel ledit échange de cations s'effectue dans une colonne échangeuse de cations Mono S.

9. Neurotrophine hétérodimère isolée et purifiée, comprenant une unité monomère d'une première neurotrophine et une unité monomère d'une seconde neurotrophine.

10. Neurotrophine hétérodimère selon la revendication 9, dans laquelle lesdites première et seconde unités monomères sont issues d'une neurotrophine choisie parmi NGF, BDNF, NT-3, NT-4 et des chimères desdites neurotrophines.

11. Composition pharmaceutique comprenant une neurotrophine hétérodimère selon la revendication 9 ou 10, conjointement avec un véhicule ou diluant pharmacologiquement acceptable.

12. Neurotrophine hétérodimère selon la revendication 9 ou 10, pour utilisation dans le traitement de l'organisme humain ou animal pour la thérapie ou dans un procédé de diagnostic.

13. Neurotrophine hétérodimère selon la revendication 12, pour utilisation dans le traitement d'une lésion du système nerveux ou le traitement d'états congénitaux ou de troubles neurodégénératifs ou dans le traitement de maladies ou de troubles impliquant des cellules striatales, ou dans le traitement de lésion ou de dégénérescence de cellules striatales ou de l'hippocampe ou dans le traitement de troubles des neurones moteurs.

14. Utilisation d'une neurotrophine hétérodimère selon la revendication 9 ou 10, dans la fabrication d'un médicament destiné à l'utilisation dans le traitement d'une lésion du système nerveux ou le traitement d'états congénitaux ou de troubles neurodégénératifs ou dans le traitement de maladies ou de troubles impliquant des cellules striatales, ou dans le traitement de lésion ou de dégénérescence de cellules striatales ou de l'hippocampe ou dans le traitement de troubles des neurones moteurs.
